# EUROPÄISCHE PATENTSCHRIFT

(11) **EP 1 364 948 B1**
(45) Veröffentlichungstag und Bekanntmachung des Hinweises auf die Patenterteilung: **10.11.2010**
(21) Anmeldenummer: 03011554.7
(22) Anmeldetag: 21.05.2003
(51) Int. Cl.: C07D 339/04, C07F 9/10, C08G 65/32, G01N 33/543

(54) **Biomimetische Systeme aus Lipidmembranen gebunden an ein Substrat**
Biomimetic system of lipid membranes bonded to a substrate
Système biomimétique issu de membranes lipidiques liées à un substrat

(30) Priorität: 22.05.2002 DE 10222588
(43) Veröffentlichungstag der Anmeldung: 26.11.2003
(73) Patentinhaber: Knoll, Wolfgang, 1220 Wien (AT)
(72) Erfinder: Schiller, Stefan, 65375 Oestrich-Winkel (DE); Naumann, Renate, 64283 Darmstadt (DE); Knoll, Wolfgang, 55124 Mainz (DE)
(74) Vertreter: Weiss, Wolfgang

(56) Entgegenhaltungen:
- WO-A-93/21528
- WO-A-94/07593
- WO-A-97/43274
- STEFAN M. SCHILLER: "ARCHAEA ANALOGUE THIOLIPIDS FOR TETHERED BILAYER LIPID MEMBRANES ON ULTRASMOOTH GOLD SURFACES." ANGEWANDTE CHEMIE. INTERNATIONAL EDITION., Bd. 42, Nr. 2, 2003, Seiten 208-211, XP002268902 VERLAG CHEMIE. WEINHEIM., DE ISSN: 0570-0833
- BERNHARD SCHUSTER: "NEW METHOD FOR GENERATING TETRAAETHER LIPID MEMBRANES" LANGMUIR, Bd. 19, Nr. 6, 18. März 2003 (2003-03-18), Seiten 2392-7, XP002268903 USA

## Beschreibung

Die Erfindung betrifft mit einem hydrophilen Linker funktionalisierte Lipide, die eine Stützungsgruppe zur Bindung an eine Oberfläche enthalten. Weiterhin betrifft die Erfindung selbstassemblierte Monoschichten der Lipide auf Substraten, insbesondere auf ultraglatten Goldsubstraten. Die Lipide und diese Lipide enthaltende Lipidmono- oder Doppelschichten können zur Herstellung von biomimetischen gestützten Membransystemen verwendet werden. Diese Systeme können gegebenenfalls nach Einbau von funktionellen Molekülen, wie etwa membranassoziierten Proteinen, für Anwendungen, wie etwa Modellsysteme zur Untersuchung von biologischen Membranen, Screeningverfahren, Sensoren und bioelektronischen Vorrichtungen, wie etwa Biocomputern, eingesetzt werden.

Die Beantwortung biologisch-medizinischer Fragestellungen ist in Zeiten des rasanten Fortschritts nicht nur im Bereich der Entschlüsselung des Erbguts kompletter Lebewesen (Genomik) und dem mit der Verifizierungung der "Code"-Funktionsbeziehungen zwischen Genom und Proteom befassten Gebiet der Proteomik in verschiedenen Bereichen der Analytik an limitierende Faktoren gestoßen. Auch in der Erforschung von Zell-ZellWechselwirkungen und Membranproteinen ist man im wachsenden Maß auf leistungsfähige Modellsysteme angewiesen, da deren Analyse in nativer Umgebung oft nur unter schwierigen Bedingungen, z.B. mit Methoden wie der Patch-Clamp Technik, möglich ist, welche sich für einen Einsatz im größeren Umfang (Standardanalysen und Meßsysteme), nicht eignen. Eine Anwendung von biologischen Modellmembransystemen, welche nicht notwendigerweise in erster Linie von deren strikten elektrischen Eigenschaften, sondern eher von ihrer Fluidität und chemischen Beschaffenheit abhängt, besteht in der Untersuchung der gerade besprochenen Vorgänge im Zusammenhang mit deren Einfluß auf Zell-ZellWechselwirkungen und (neuro)degnerativen Zellveränderungen, welche sich auch in veränderten Glycolipidstrukturen auf der Zelloberfläche bemerkbar machen (Kolter, Sandhoff, Angew. Chem. Int. Ed. 1999, 38, 1532-1568) oder sich beim Auftreten veränderter Glycopeptidstrukturen (z.B. bei Mucinen, welche eine wichtige Rolle bei Zellwechselwirkungen spielen (Schiller, S., Diplomarbeit, Mainz, 1998)), zeigen.

Die Suche nach biomimetischen Systemen, welche die Erforschung solcher Systeme oder deren Einsatz in der Analytik ermöglicht, stellt hohe Anforderungen an das verwendete Modellsystem. Eine Grundanforderung ist die größtmögliche Übereinstimmung von natürlichem Vorbild und dem biomimetischen Modell, welches das natürliche Vorbild in seinen für die entsprechende Fragestellung wesentlichen Eigenschaften nachzuahmen in der Lage sein muss.

Eines der wichtigsten biomimetischen Systeme besteht aus einer der biologischen Membran ähnlichen Lipiddoppelschicht, die auf einem elektrisch leitenden, planaren Substrat aufgebracht ist. Ein solches System ermöglicht die Untersuchung von membranassoziierten Biomolekülen (z.B. H⁺-ATPase, Cytochromoxidase, lonenkanäle etc.), die mit elektrischen Vorgängen zusammenhängen bzw. von ihnen kontrolliert werden und daher in der Biosensorik und im Drug Screening Verwendung finden.

Beschreibungen einer Vielzahl von Anwendungen, ihrer biophysikalischen Eigenschaften und unterschiedlichsten chemischen Zusammensetzungen bezüglich der die Membran stützenden Molekülteile und Variationen im Lipidteil (von gesättigten bis zu ungesättigten Alkylketten unterschiedlicher Kettenlänge, in monomerer, dimerer oder mehrfach auftretender Form, welche zusätzlich noch mit hydrophoben Seitengruppen versehen sein können und verschiedene polare, diese verbindende Kopfgruppen enthalten können) und deren Anbindung an die meist polare eben genannten Stützungsgruppe (Tether) finden sich bei Ringsdorf et al., Angew. Chem. Int. Ed., 1988, 27, 113-158; Lang, et al., Langmuir, 1994, 10, 197; Sackmann, Science, 1996, 271, 43-48; Guidelli et al., J. Electroanal. Chem., 2001, 504, 1-28 und Knoll et al., Mol. Biotechnol., 2000, 74, 137-158.

Die funktionelle Anbindung der Lipidmembran an ein festes Substrat über einen flexiblen, polaren (hydrophilen) Zwischenteil ist ein seit Jahren populärer Ansatz (Sackmann, siehe oben) mit folgenden Vorteilen: Zum einen wird über sie eine robuste Anbindung der empfindlichen Lipiddoppelschicht an das feste Substrat erreicht, welche durch ihre Planarität Zugang zu einer Reihe sensitiver elektrischer und oberflächenanalytischer Methoden erschließt, zum anderen entkoppelt sie die Membran von der Oberfläche. Die Entkopplung von der Oberfläche führt zu einer Unterdrückung der auf Metalloberflächen auftretenden, für die Membran negativen hydrophoben Wechselwirkung, und vergrößert das cytosolanaloge Volumen zwischen Membran und Substratoberfläche, was besonders bei volumenabhänigen Transportprozessen, als auch zur Erzeugung von fluiden Membranen von grundlegender Bedeutung ist.

Die zur Anwendung auf Membransysteme geeigneten Messmethoden umfassen bei den elektrischen Methoden z.B. Impedanz-Spektroskopie (IS), Voltametrie, und andere potentiometrische und amperometrische Verfahren, wie z.B. die Chrono-Coulometrie. Die oberflächenanalytischen Verfahren setzen sich aus einer Reihe von Methoden, von denen viele besonders auf planare Metallfilme als proximale Schicht vor der Modifizierung mit dem zu analysierenden System angewiesen sind, zusammen. Sie erlauben oft eine Analyse bis in submolekularen Größenordnungenen, welche nur durch solche festkörpergestützte Membranen zugänglich ist. Hierzu zählt zum Beispiel die Oberflächen-Plasmonen-Resonanzspektroskopie (OPR, SPR oder auch SPS genannt) zur Bestimmung der Schichtdicke (Brechungsindex) der auf dem Substrat (z.B. Au- oder Ag-Filmen) aufgebrachten Verbindungen. Zur Charakterisierung des beschriebenen Systems können aber noch eine große Zahl anderer, zum Teil sehr empfindlicher Meßverfahren angewandt werden. Hierzu gehören Methoden wie Ellipsometrie, Fluoreszenzmethoden, interne Reflektometrie, Lichtstreumethoden, Röntgen- oder Neutronenstreuung, oberflächenakustische Verfahren, Ausnutzung thermischer Effekte (Änderungen der Temperatur oder des Wärmeflusses), Messung der Massen oder Dichtenänderungen, welche z.B. mit der Quarzmikrowaage ermittelt werden können, Messung der Änderung der Membranphase, Radioimmunassays oder Enzym gebundene Assays.

Die Verwirklichung eines Modellsystems, welches die Anwendung der oben genannten Methoden erlaubt und andererseits die Voraussetzung für eine funktionelle biologische Modellmembran mit den notwendigen Eigenschaften gibt, ist ein Problem, welches höchste Anforderungen an die molekularen Eigenschaften der Membranbestandteile, deren Oberflächenanbindung, die sie umgebenden Moleküle, die Eigenschaften und Güte der Oberfläche und die anzuwendenden Messmethoden stellt.

Aus dem Stand der Technik sind zahlreiche Beispiele für gestützte Membransysteme bekannt. So können beispielsweise Membransysteme auf Basis von thiolmodifizierten Oligopeptiden und Thiollipopeptiden eingesetzt werden (siehe z.B. Bunjes et al., Langmuir, 1997, 13, 6188-6194; Naumann et al., Bioelectrochem. Bioenerg. 1997, 42, 241-247; Schmidt et al., Biosensens. Bioelectron. 1998, 13, 585-591; Naumann et al., Biosens. Bioelectron. 1999, 14, 651-662 sowie WO 96/18645 und WO 99/20649). Von Peggion et al., Langmuir, 2001, 17, 6585-6592, sind ebenfalls Oligopeptide als membranstützende Komponenten beschrieben, welche mit polaren Triethylenglykolseitenketten versehen sind und eine hydrophile Monoschicht ausbilden.

Mit Polyethylenglykol hydrophil gestützte Membransysteme sind bei Lang et al., Langmuir, 1994, 10, 197-210, Heyse et al., Biochem. Biophys. Acta 1998, 85507, 319-338, EP-A-0 441 120 und EP-A-0 637 384 beschrieben. Ein weiteres, mit einer kurzen Oligoethylenglykolkette gestütztes Membransystem, welches Cholesterin als hydrophobe Domäne aufweist, ist von Williams et al., Langmuir 1997, 13, 751-757 und Jenkins et al., Langmuir, 1998, 14, 4657, beschrieben.

Weitere Membransysteme sind von Cornell et al., Nature 1997, 387, 580-583, Raguse et al., Langmuir 1998, 14, 648-659, Krishna et al., Langmuir 2001, 17, 4858-4866 sowie in WO 89/01159, WO 90/02327, WO 94/07593, WO97/01092, US 5,753,093 und US 5,783,054 beschrieben. Die zum Aufbau der Membran verwendeten Komponenten bestehen aus verschiedenen Monophytanyl/Monophytanoyl-Oligoethylenglycol-Thiolen/Disulfiden, polaren lateralen Spacermolekülen und einem membrandurchspannenden Thiolipid. In die Membran können Funktionalisierungsmoleküle, wie z.B. Gramicidin, eingebaut werden. Die Membran wird nach Behandeln einer Goldoberfläche mit einer ethanolischen Lösung der Einzelkomponenten durch Spülen mit Puffer in situ erzeugt.

In WO 97/43274 werden Verbindungslipide beschrieben, mit welcher eine Membran mit einer Elektrode verbunden werden kann. Die Membran wird hierbei entweder zum Teil oder vollständig aus dem Verbindungslipid gebildet. Die Verbindungsiipide bestehen aus einer polaren Kopfgruppe, einer hydrophoben Region, einer hydrophilen Region und einer Befestigungsgruppe, mit welcher das Lipid an die Elektrodenoberfläche befestigt werden kann. Die hydrophobe Region kann zumindest zur Hälfte die Membran durchspannen.

Nachteile der oben genannten Membransysteme bestehen darin, dass oftmals nur ungenügende elektrische Eigenschaften, insbesondere hinsichtlich der Kapazität und des Widerstands bei Bindung an eine elektrisch leitfähige Substratoberfläche erhalten werden. Einige der aus dem Stand der Technik bekannten Membransysteme weisen darüber hinaus eine unzureichende chemische Stabilität auf. Somit bestand eine der vorliegenden Erfindung zugrunde liegende Aufgabe darin, neue Membransysteme bereitzustellen, bei denen die oben genannten Nachteile zumindest teilweise beseitigt sind. Insbesondere sollten die erfindungsgemäßen Membransysteme verbesserte elektrische Eigenschaften gegenüber dem Stand der Technik aufweisen.

Diese Aufgabe wird gelöst durch die Bereitstellung von neuen Verbindungen der allgemeinen Formeln (la) und (Ib): 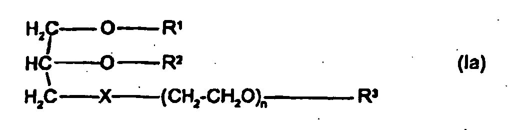 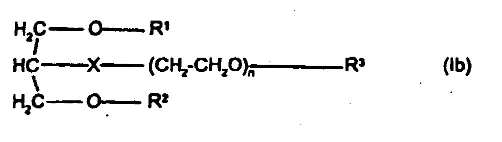 worin mindestens einer von R¹ und R² jeweils unabhängig ein gesättigter oder ungesättigter Kohlenwasserstoffrest oder ein Acylrest mit einer Kettenlänge von 10-22 C-Atomen ist, der gegebenenfalls durch eine oder mehrere Seitengruppen, z.B. C₁-C₄-Alkylgruppen und insbesondere Methylgruppen, substituiert sein kann, und der andere Wasserstoff, ein C₁-C₉-Kohlenwasserstoffrest oder ein Rest, umfassend eine Phospholipid-, Carboxyl-, Carbonyl-, SO-, SO₂-, Amid-, Amino- oder Thiolgruppe mit oder ohne einem C₁-C₉-Kohlenwasserstoffrest sein kann,
- n: eine ganze Zahl von 1-100 ist,
- R³: ein eine Stützungsgruppe enthaltender Rest ist und
- X: eine Verknüpfungsgruppe ist, die aus O, S oder NR ausgewählt wird,
wobei R für H oder einen C₁-C₄-Alkylrest steht, die zur Verankerung von Membranen an einer Substratoberfläche, insbesondere einer elektrisch leitfähigen Substratoberfläche, geeignet sind.

Dabei stehen die Verbindungen (Ia) sowohl für die 1,2 sn- als auch für die 2,3 sn-Lipide nach der für Lipide geltenden stereochemischen Nomenklatur (sn).

Vorzugsweise ist zumindest einer von R¹ und R² ein gesättigter oder ungesättigter Kohlenwasserstoff- oder Acylrest, der durch eine oder mehrere Methylgruppen substituiert ist. Besonders bevorzugt sind R¹ und R² derartige Kohlenwasserstoff- oder Acylreste. Die Kettenlänge von R¹ und R² beträgt vorzugsweise 12-20 C-Atome und besonders bevorzugt 13-18 C-Atome. Beispiele für geeignete Kohlenwasserstoff- oder Acylreste sind Phytanyl Phytanoyl, Lauryl, Lauroyl, Tridecanyl, Tridecanoyl, Myristyl, Myristoyl, Pentadecanyl, Pentadecanoyl, Palmityl, Palmitoyl, Oleyl, Oleoyl, Linoleyl, Linoleoyl, Arachidonoyl, Docosahexaenyl, Docosahexaenoyl etc. Besonders bevorzugt sind Kohlenwasserstoffreste, die an den Träger über eine Ethergruppe gebunden, wie Phytanyl.

Die Verknüpfungsgruppe X ist vorzugsweise O.

Die Verbindungen der allgemeinen Formeln (Ia) und (Ib) sind vorzugsweise Glycerinderivate mit insbesondere 2 Kohlenwasserstoff- oder/und Acylresten und einem zur Verankerung an ein Substrat dienenden Rest, der eine Oligoethylenoxidgruppe mit beispielsweise 1-100 Ethylenoxideinheiten enthält.

In einer besonders bevorzugten Ausführungsform wird die Verbindung (I) aus 2,3-Di-O-phytanyl-sn-glycero-1-tetraethylenglycol-DL-α-liponsäureester und optischen Isomeren davon ausgewählt. Weitere Beispiele für geeignete Verbindungen sind Diphytanyl/oyl-phosphatidyl-tetraethylenglycolliponsäureester, entsprechende Verbindungen, bei denen die Liponsäurestruktur anstelle der Esterverknüpfung eine Ether- oder Kohlenstoff-Kohlenstoffverknüpfung trägt, und die Stereochemie am Glycerinbaustein auch die entsprechenden 1,2- und 1,3-Di-O-phytanylverknüpfungen enthält. Denkbar ist ebenso die Wahl eines anderen Trägers der Phytanylgruppen anstelle des Glycerinbausteins, wie z.B. Tris[2-Amino-2-hydroxymethyl-1,3-propandiol], welcher eine zusätzliche polare Hydroxylgruppe trägt, oder in der Lage ist, eine dritte Phytanylgruppe aufzunehmen.

Die erfindungsgemäßen Verbindungen (I) zeichnen sich dadurch aus, dass sie zwei unpolare Lipidgruppen enthalten, die über eine Ether- oder Esterbindung an einen Trägerbaustein, z.B. einen D-Glycerinbaustein, gebunden sind. An der dritten Funktionalität, z.B. an einer Hydroxygruppe, enthält der Trägerbaustein eine Oligoethylenglycolkette, die vorzugsweise ebenfalls über eine Ethergruppen gebunden ist. Aufgrund ihrer Struktur besitzen die erfindungsgemäßen Verbindungen eine geringe Hydrolyseempfindlichkeit, die sich bei Langzeitanwendungen als sehr vorteilhaft erweist. Als Stützungsgruppe wird vorzugsweise eine Disulfidgruppe, besonders bevorzugt eine cyclische Disulfidgruppe, verwendet. Günstigerweise sind beide Schwefelatome des Disulfids kovalent mit dem polaren Oligoethylenoxidrest verbunden.

Die Verbindungen (I) können auf einer Substratoberfläche über die Stützungsgruppe verankert werden. Diese Verankerung ist vorzugsweise eine adsorptive Bindung, durch die eine laterale Beweglichkeit einzelner Moleküle der Verbindungen (I) auf der Substratoberfläche gewährleistet bleibt. Zusätzlich zu den Molekülen der Verbindungen (I) können auf der Substratoberfläche noch Spacermoleküle, d.h. Moleküle mit einer Stützungsgruppe und gegebenenfalls einer hydrophilen Gruppe, gebunden werden, um eine Verdünnung der Moleküle der Verbindungen (I) zu erreichen. Beispiele für geeignete Spacermoleküle mit S-haltigen Stützungsgruppen sind Liponsäure, Thioethanol, Thiodiglycolsäure, Cystein, Thioessigsäure, Dimercaptobernsteinsäure, Hydroxy/Carboxy-Alkylthiole und Disulfide einer Kettenlänge von 1-12 Kohlenstoffatomen, und analoge Dialkylsulfide (Thioether), aber auch hydrophobe Verbindungen, wie Alkylthiole und Disulfide einer Kettenlänge von 1-12 Kohlenstoffatomen, und analoge Thioether, Phenylthiol, Bisphenylthiol und andere. Das molare Verhältnis von Molekülen der Verbindungen (1) zu Spacermolekülen liegt vorzugsweise im Bereich von 100:0,05 bis 0,05:100, besonders bevorzugt im Bereich von 0,1:100 bis 100:1 und am meisten bevorzugt von 15:100 bis 70:1. Selbstverständlich können aber auch reine Stützlipidschichten eingesetzt werden.

Ein weiterer Aspekt der Erfindung ist eine gestützte biomimetische Membran, umfassend eine Lipidschicht mit mindestens einer Verbindung der allgemeinen Formel (I) als Stützungslipid zur Verankerung auf einem Substrat. Die Lipidschicht kann eine Monoschicht oder - wenn zusätzliche mobile Lipide vorhanden sind - eine Doppelschicht umfassen.

Das Substrat weist vorzugsweise eine elektrisch leitfähige Oberfläche, beispielsweise eine Metalloberfläche, insbesondere eine Edelmetalloberfläche, wie etwa Gold, Silber oder Platin, auf. Besonders bevorzugt ist eine Goldoberfläche.

Weiterhin besonders bevorzugt ist ein Substrat mit einer ultraglatten Oberfläche, die eine Oberflächenrauheit in oder unterhalb der Größenordnung einer Moleküldimension aufweist, z.B. maximal 2-3 nm, bevorzugt unter 2 nm, besonders bevorzugt unter 1 nm und im gezeigten Beispiel 0,5 nm. Ein Beispiel für eine derartige ultraglatte Oberfläche ist eine ultraglatte Goldoberfläche, die durch Templatabformung von Goldoberflächen zugänglich ist (Wagner et al., Langmuir, 1995, 11, 3867-3875). Bei Vorliegen einer ultraglatten Oberfläche ist eine optimale supramolekulare Anordnung der einzelnen funktionellen Abschnitte der Verbindungen (I) gegeben, so dass eine besonders günstige hydrophobe Wechselwirkung der Kohlenwasserstoffketten zustande kommen kann.

Aus einer Monoschicht, die Verbindungen der allgemeinen Formel (I) enthält, kann durch Zugabe von mobilen Lipiden, beispielsweise durch Vesikelfusion, eine gestützte Lipiddoppelschicht gebildet werden. Beispiele für geeignete mobile Lipide sind Phospholipide, wie etwa 1,2-Di-Ophytanyl-sn-glycero-3-phosphocholin (DphyPC), 1,2-Di-O-phytanoyl-snglycerophosphocholin, 1,2-Di-O-phytanyl-sn-glycerophosphoethanolamin, 1,2-Di-O-phytanoyl-snglycerophosphoethanolamin, entsprechende 1,2- oder 2,3-Diphytanyl- oder Diphytanoylderivate sowie Mischungen von mehreren der genannten Verbindungen oder andere Phospholipide, wie etwa Ei-Phosphatidylcholin, DMPC (Dimyristoylphosphatidylcholin),DMPE (Dimyristoylphosphatidylethanolamin), Lecithine und nicht phosphorhaltige Lipide in gesättigter und ungesättigter Form, versehen mit gleichen oder unterschiedlich substituierten oder nichtsubstituierten Alkylketten mit vorzugsweise 8-22 Kohlenstoffatomen, insbesondere mit zwei Phytanyl-oder/und Phtanoylgruppen, welche an Glycerin über Ether- oder/und Esterbindungen verknüpft sind, Glycolipide, quarternäre Ammoniumverbindungen mit zwei C₁-C₄-Alkylgruppen und zwei C₈-C₁₈-Alkylketten, tertiäre Amine mit zwei C₈-C₁₈-Alkylketten und einer C₁-C₄-Alkylkette oder einem Wasserstoffatom anstelle der C₁-C₄-Alkylkette, Steroide oder chemisch ähnliche Verbindungen, welche sich vom Cholestangerüst ableiten lassen oder Isoprenoide, sowie Lipidmischungen, bestehend aus den genannten Verbindungen, wobei die Lipide gegebenenfalls polare Kopfgruppen, wie Hydroxyl, Carboxyl, Phosphorsäureester und deren Derivate, Schwefeloxidderivate unterschiedlicher Oxidationsstufe des Schwefels, und natürlich vorkommende Gruppen, wie Choline, Ethanolamine, Inositole, Glycerine, Aminoglycerine (in Sphingosinen) und Aminosäuren und deren neutrale, kationische oder anionische Formen oder Derivate enthalten können. Bei Verwendung von Vesikeln mit Membranen, in denen funktionelle Moleküle, beispielsweise Membranproteine integriert sind, können diese bei der Vesikelfusion direkt in die gestützte Membran eingebaut werden.

Die Präparation der Lipiddoppelschicht kann darüber hinaus auch durch LB (Langmuir-Blodgett)-Übertrag von Lipidschichten, spontane Doppelschichtbildung, durch Verdünnen einer z.B. ethanolischen Lipidlösung mit Wasser oder Pufferlösung oder durch Umkehrosmose und Verdünnen vesikulärer Lösungen erfolgen.

Ein weiterer Vorteil der erfindungsgemäßen gestützten Membran besteht in ihrem einfachen Aufbau, da sie aus einer Monoschicht mit nur einer einzigen Verbindung (I) aufgebaut werden kann. Die Verwendung von synthetisch aufwendigen und dadurch teuren membrandurchspannenden Lipiden ist nicht erforderlich. Außerdem kann ein mit einer Monoschicht modifiziertes Substrat, beispielsweise ein Goldträger, ohne Qualitätsverlust trocken gelagert werden und bei Bedarf einfach durch Vesikelfunktion oder eine andere oben beschriebene Methode in eine funktionsfähige biologische Modellmembran umgewandelt werden. Im Gegensatz dazu erfordert die Lagerung von Systemen des Standes der Technik besondere Maßnahmen, da dort nur komplette Modellmembranen hergestellt und aufbewahrt werden können, welche ohne Zugabe spezieller Verbindungen beim Trocknen sofort desintegrieren. Außerdem kann bei derartigen Systemen schon nach kurzer Aufbewahrung ein merklicher Qualitätsverlust auftreten.

Die erfindungsgemäßen gestützten Membranen weisen hervorragende elektrische Eigenschaften auf. So können sie eine Kapazität von 0,4 bis 0,7 µF/cm², vorzugsweise von 0,4 bis 0,6 µF/cm², aufweisen. Der Widerstand ist vorzugsweise von mindestens 1 MΩ·cm², besonders bevorzugt mindestens 5 MΩ·cm², beispielsweise im Bereich von 6-24 MΩ·cm².

In die Lipidmembran können funktionelle Moleküle, wie etwa Proteine, beispielsweise membranassoziierte Proteine, wie H⁺-ATPasen, Cytochromoxidasen, lonenkanäle etc., die mit elektrischen Vorgängen zusammenhängen, andere Membranproteine, lichtabhängige Proteine, wie Bakteriorhodopsin, Antibiotika, Membranrezeptoren und Liganden, z.B. Peptide oder Proteine oder zusätzliche kohlenhydrat- oder/und lipidhaltige Strukturen, oder aus Heterozyklen bestehende Strukturen oder Kombinationen davon eingebaut werden.

Die erfindungsgemäßen gestützten Membranen können für eine Vielzahl von Anwendungen, beispielsweise in der Biosensorik und im Wirkstoffscreening, z.B. zur Identifizierung oder/und Charakterisierung pharmakologischer Wirkstoffe, eingesetzt werden. Weiterhin können die gestützten Membranen zu Analyse von Biomolekülen, beispielsweise von membranassoziierten Biomolekülen, ausgewählt aus Proteinen, Antibiotika, etc., dienen. Außerdem können die Membranen aufgrund ihres hohen Widerstandes und ihrer effektiven Ladungsseparation für elektrotechnische Anwendungen, insbesondere als elektrische Dichtungsmaterialien, dienen. Sie können beispielsweise in bioelektro- mechanischen Mikro- und Nanovorrichtungen (Bio-MEMS/NEMS) zur elektrischen Abdichtung bzw. Einbettung von makroskopischen oder/und molekularen elektrischen Bauteilen und für molekulare Drähte (Aviram und Rathner, Chem. Phys. Let. 1974, 29, 257) Verwendung finden, insbesondere deshalb, weil sich ihre Dicke auf molekularer Größenordnung befindet. Darüber hinaus sind die Membranen auch durch ihr Kompartimentierungsvermögen auch in der Lage, als Systeme zu dienen, welche z.B. als Nahtstelle für biomechanische Fragestellungen im Bereich von ATP-abhängigen molekularen Motoren, wie z.B. Myosin, Kinesin und Dynein (Taylor et al., Nanotechnology, 1999, 10, 237-243) Systemkomponenten liefern können, vor allem wenn man sie mit ATP-liefernden Membransystemen oder anderen funktionellen Systemen, z.B. lichtabhängigen Proteinen, wie Bakteriorhodopsin, kombiniert.

Weiterhin soll die Erfindung durch die nachfolgenden Abbildungen und Beispiele erläutert werden.

### Abbildung 1:

2,3-Di-O-phytanyl-sn-glycero-1-tetraethylenglycol-DL-α-liponsäureester (DPTL)

### Abbildung 2:

1,2-Di-O-phytanoyl-sn-glycero-phosphocholin (DPhyPC)

### Abbildungen 3 und 4:

Rasterkraftmikroskopische Aufnahmen von Goldsubstraten

### Abbildung 5:

Kinetik einer Vesikelfusion mit einer DPTL-Monoschicht

### Abbildung 6:

Oberflächen-Plasmonenresonanz(SPR)-Spektrum einer DPTL-Schicht vor und nach einer Vesikelfusion

### Abbildung 7:

Impendanz(IS)-Spektrum einer DTPL-Schicht vor und nach einer Vesikelfusion

### Abbildung 8:

Spektren einer DTPL-Lipiddoppelschicht vor und nach Einbau von Valinomycin

### Beispiele

### Beispiel 1:Synthese von 2,3-Di-O-phytanyl-sn-glycero-tetraethylenglycol-DL-α-liponsäureester

Verbindungen gemäß Formel (I), z.B. 2,3-Di-O-phytanyl-sn-glycero-1-tetraethylenglycol-DL-α-liponsäureester und deren Ausgangsstoffe können nach bekannten Methoden hergestellt werden, wie sie in der Literatur (z.B. Standardwerken wie Houben Weyl, Methoden der organischen Chemie, Georg Thieme-Verlag, Stuttgart) beschrieben sind, und zwar unter Reaktionsbedingungen, die für die genannten, auch die hier nicht näher erwähnten Varianten Gebrauch machen.

D-Mannit wird unter Standardbedingungen, beschrieben von Baer, J. Amer. Chem. Soc. 1945, 67, 338, zur diisopropylidengeschützten Verbindung umgesetzt (1,2;5,6- Diisopropyliden-D-Mannit). Diese wird mit Natriumperiodat bei 0 °C in Wasser oxidativ zum Aldehyd gespalten und die Lösung nach vollständigem Umsatz mit Ethanol aufgearbeitet, der entstehende Niederschlag abgetrennt und die verbleibende Lösung mit Natriumborhydrid, suspendiert in Wasser, im Eisbad versetzt und der Aldehyd zum 2,3-Isopropyliden-D-glycerin reduziert. Die Reaktion wird über Nacht gerührt und am nächsten Morgen mit Essigsäure auf pH 7 eingestellt. Anschließend wird das organische Lösungsmittel destillativ entfernt. Die wässrige Phase wird mit Methylenchlorid extrahiert und die organische Phase mit Natriumsulfat/Natriumcarbonat getrocknet. Nach Entfernen des Lösungsmittels wird der isopropylidengeschützte Glycerinbaustein erhalten. Der isopropylidengeschützte Glycerinbaustein wird in Chloroform aufgenommen und mit Molsieb (3 Å) getrocknet.

Alle weiteren Schritte bis zur Aufarbeitung werden unter Argonatmosphäre durchgeführt. Zur Lösung wird p-Toluolsulfonsäurechlorid gegeben und nach 15 min Pyridin (über Kaliumhydroxid getrocknet) zugetropft. Das Eisbad wird entfernt und der Ansatz über Nacht gerührt. Der Ansatz wird mit Wasser und gesättigter Natriumchloridlösung extrahiert und die organische Phase über Natriumsulfat getrocknet und das Lösungsmittel destillativ entfernt. Das erhaltene Rohprodukt wird durch Flashchromatographie an Kieselgel mit Petrolether/ Essigsäureethylester-Mischungen als mobile Phase gereinigt.

Tetraethylenglycol wird in Tetrahydrofuran (über Calciumhydrid getrocknet) gelöst und mit Molsieb (3 Å) getrocknet (der Kolben mit Argon gefüllt). Das Molsieb wird entfernt, und das Tetraethylenglycol mit einem Moläquivalent Natriumhydrid deprotoniert. Dann wird der tosylierte, isopropylidengeschützte Glycerinbaustein zugetropft, anschließend Dimethyaminopydridin zugegeben und erst 6 h bei Raumtemperatur, anschließend 3 Tage bei 60 °C unter Argonatmosphäre gerührt. Der Ansatz wird über Celite®-Filtergel filtriert, das Lösungsmittel destillativ entfernt und das Rohprodukt mit Dichlormethan verdünnt und mit Wasser extrahiert. Die organische Phase wird mit Natriumsulfat getrocknet und das Lösungsmittel destillativ entfernt. Die erhaltene Produktphase wird in trockenem Dimethylformamid (über Calciumhydrid destilliert) aufgenommen, auf -2,5 °C abgekühlt und mit Natriumhydrid deprotoniert. Anschließend wird bei 0 °C Benzylbromid zugetropft, wobei die Temperatur unter 10°C gehalten wird. Nach vollendeter Zugabe wird das Kältebad entfernt und der Ansatz bei Raumtemperatur zwei Tage unter Argonatmosphäre gerührt. Der Ansatz wird über Celite®-Filtergel filtriert und das Lösungsmittel destillativ entfernt. Das Rohprodukt wird in Dichlormethan aufgenommen, mit Wasser extrahiert und durch Flashchromatographie an Kieselgel mit einer mobilen Dichlormethanphase gereinigt. Der erhaltene Benzyl-tetraethylenglycol-2,3-isopropyliden-D-glycerinbaustein wird mit Tetrahydrofuran/Wasser versetzt, und die Isopropylidenschutzgruppe mit Trifluoressigsäure bei 40 °C 36 h abgespalten. Die Reaktionsmischung wird mit Natriumcarbonat neutralisiert, und nach weiterer Wasserzugabe mit Dichlormethan extrahiert. Die organische Phase wird getrocknet, das Lösungsmittel destillativ entfernt und das erhaltene Rohprodukt durch Flashchromatographie an Kieselgel mit einer mobilen Phase aus Essigsäureethylester und Aceton mit zunehmendem Aceton-Gradienten gereinigt.

Für die Modifizierung des Produkts mit den zwei Phytanylresten wird zuerst Phytol mit Platinoxid in Methanol bei 40 °C und 70 bar Wasserstoffdruck 2 Tage zu Phytanol hydriert. Das erhaltene Rohprodukt wird durch Gelfiltration an Kieselgel mit Hexan gereinigt. Das erhaltene Phytanol wird in Dichlormethan gelöst und mit Triphenylphosphin und N-Bromsuccinimid bei einer Temperatur von weniger als 30°C über Nacht bromiert. Aus dem Rohprodukt wird das Dichlormethan destillativ entfernt, der Rückstand mit Hexan extrahiert und über eine Glasfritte filtriert. Das Hexan wird destillativ entfernt und das Phytanbromid enhaltende Rohprodukt mit Hexan an Kielselgel durch Flashchromatographie gereinigt.

Der mit einseitig benzylethergeschütztem Tetraethylenglycol modifizierte D-Glycerinbaustein, bei dem die 2- und die 3-Position durch Abspaltung des Isopropylidenrests mit Trifluoressigsäure entschützt wurden, wird in trockenem Dimethyformamid gelöst, mit Natriumhydrid deprotoniert und unter Argonatmospähre das Phytanbromid mit einem Tropftrichter zugegeben. Der Kolben wird unter Auschluss von Licht 4 Tage bei Raumtemperatur gerührt. Nach der Zugabe von Wasser wird mit Dichlormethan extrahiert, die organische Phase mit Natriumsulfat getrocknet und das Lösungsmittel destillativ entfernt. Das Rohprodukt wird durch Flashchromatographie an Kieselgel mit einer mobilen Petrolether/Essigsäureethylester-Phase gereinigt. Der 2,3-Di-O-phytanyl-sn- glycero-1-tetraethylenglycol-O-benzylether wird in Tetrahydrofuran/ Methanol gelöst und die Benzyletherschutzgruppe palladiumkatalysiert hydrogenolytisch bei Raumtemperatur analog bekannten Standardverfahren gespalten. Nach 2 h wird das Lösungsmittel durch Destillation entfernt und der Ansatz durch Flashchromatographie an Kieselgel mit Petrolether/Aceton als mobile Phase gereinigt.

Das erhaltene Produkt wird in trockenem Dichlormethan gelöst, in dem zuvor DL-Liponsäure mit N-(3- Dimethylaminopropyl)-N'-ethyl-carbodiimid Hydrochlorid vorgelegt und für 20 min gerührt worden ist. Zu dieser Lösung wird nach 30 min Dimethylaminopyridin gegeben und die Reaktion für 4 1/2 h gerührt. Der Ansatz wird über Celite®-Filtergel filtriert, das Rohprodukt mit Dichlormethan verdünnt und mit gesättigter Natriumhydrogencarbonatlösung und Wasser extrahiert. Die organische Phase wird mit Natriumsulfat getrocknet und das Lösungsmittel destillativ entfernt. Die Reinigung geschieht durch Flashchromatographie an Kieselgel mit Petrolether/Aceton als mobile Phase. Die Reinheit der Produkte und ihre Identität wird durch Dünnschichtchromatographie, NMR- und IR-Spektroskopie, Polarimetrie und Elementaranalysen bestätigt.

### Beispiel 2:Herstellung von Goldsubstraten (von Glimmer abgelöstem Gold)

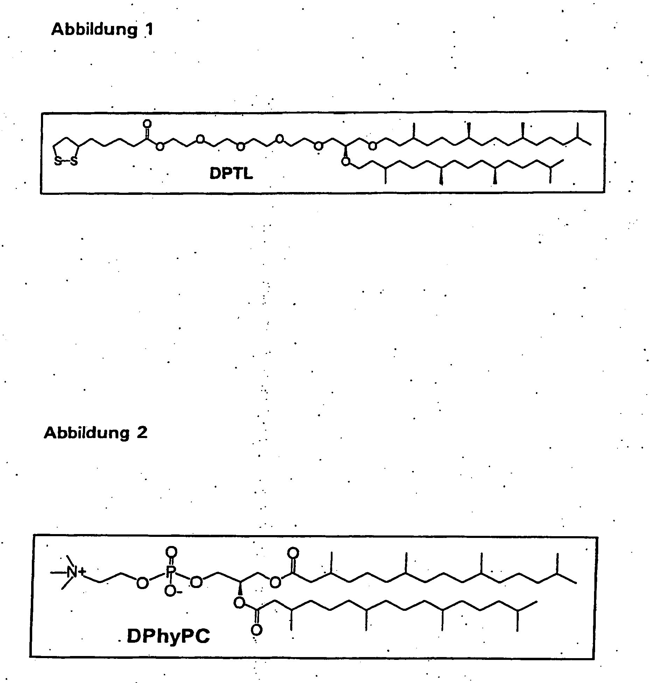

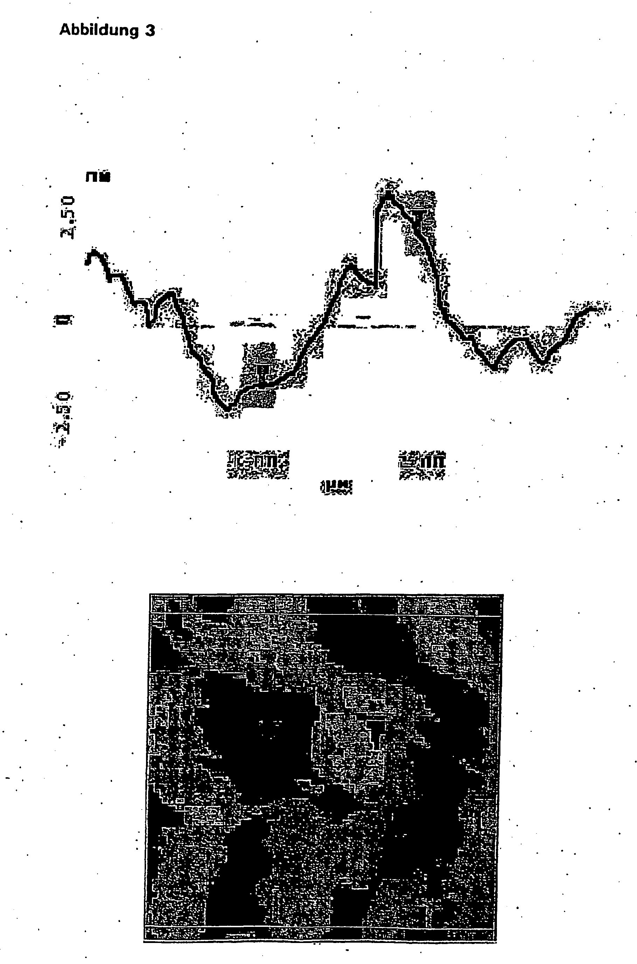

Glimmer wird unter äußersten Reinheitsbedingungen in dünne Schichten gespalten, so dass die oberste Schicht möglichst aus einer geschlossenen Kristallfläche besteht. Um anhaftendes Wasser zu entfernen, werden die Glimmerplättchen im Röhrenofen bei 650±1 °C im leichten Stickstoffstrom ausgeheizt, wobei sie an einem Stahldrahthalter in 15 s bis in die Mitte des Ofenrohrs bewegt, dann dort 30 s gehalten und in weiteren 15 s aus dem Ofen heraus bewegt werden. Danach werden sie in eine elektrothermische Bedampfungsapparatur gebracht (z.B. Elektrothermischer Evaporator, Edvards) und sofort evakuiert. Dort wird bei einem Druck von <5 x 10⁻⁶ mbar und einer Aufdampfrate von <0,1 nm/s ein plasmonenfähiger Goldfilm, z.B. 60 nm, aufgedampft. Nach dem Abkühlen werden die mit Gold bedampften Glimmersubstrate aus der Bedampfungsapparatur entnommen und sofort unter den gleichen Bedingungen wie beim Ausheizen der Glimmersubstrate (650 °C, Stickstoffstrom, 15 s, 30 s, 15 s) getempert, wobei das Gold die Kristallstruktur von Au(111) annimmt. Dies konnte durch Rasterkraftmikroskopie (AFM) nachgewiesen werden (Abb. 3).

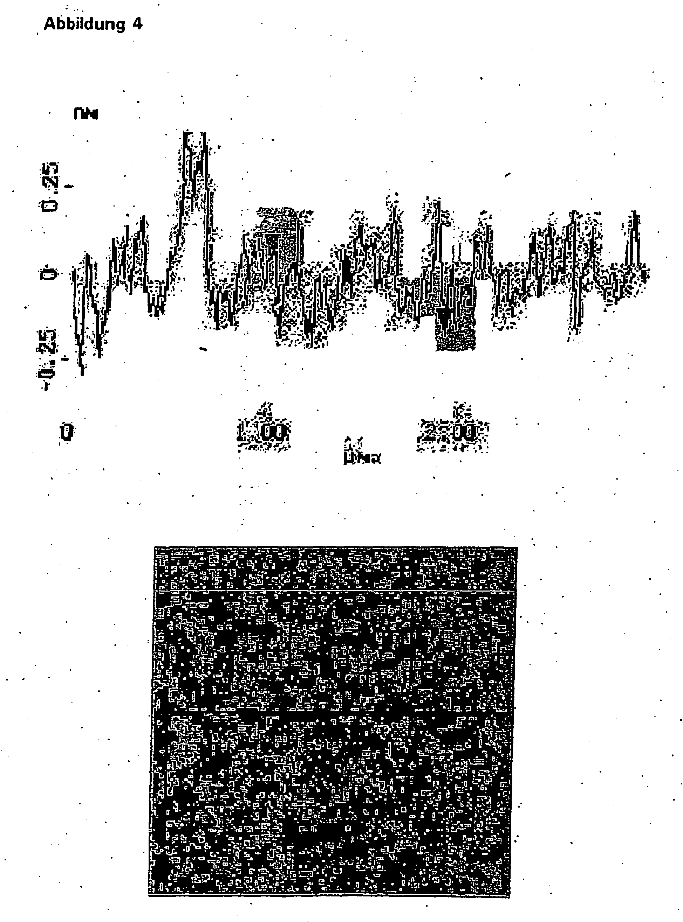

Danach werden die Glimmergoldsubstrate mit der Goldseite auf Glassubstrate (z.B. hochbrechendes Glas - LaSFN9, sofern die Substrate auch durch SPR untersucht werden sollen) aufgeklebt, wobei der Klebstoff durch kräftiges Anpressen gleichmäßig über die Fläche verteilt wird. Nach dem Trocknen bei 150 °C (60 min) werden die Substrate für 2-3 min in Tetrahydrofuran gestellt. Danach werden die Glimmerplättchen abgezogen, so dass nun das Gold mit der vorher dem Glimmer zugewandten (glatten) Seite nach oben zeigt. Die rasterkraftmikroskopische Aufnahme (Abb. 4) zeigt nun eine feinkörnigere aber glattere Oberfläche, vergleiche die Rauhigkeiten des Au(111), Abb. 3, und des TSG, Abb. 4.

Die fertigen Substrate werden günstigerweise sofort in eine Beschichtungslösung getaucht. Andernfalls können sie in reinem Ethanol aufbewahrt werden.

### Beispiel 3:Herstellung von monomolekularen Schichten (self assembled monolayers (SAM's)) von DPTL durch Selbstorganisation auf Gold

Die Goldsubstrate werden 24 Stunden lang in einer Lösung von DPTL in Ethanol (0,2 mg/ml) aufbewahrt, danach mit Ethanol gespült, im Stickstoffstrom getrocknet und bis zur Verwendung in einer Argonatmosphäre aufbewahrt.

### Beispiel 4:Herstellung von verankerten Lipiddoppelschichten (Tethered lipid bilayers) durch Vesikelfusion der Monoschicht und Messung ihrer Schichtdicken und elektrischen Eigenschaften

In einer Anlage zur kombinierten Oberflächenplasmonenresonanz-Spektroskopie (Surface plasmon resonance spectroscopy (SPR)) und Impedanzspektroskopie (IS) und der dazugehörigen Messzelle, siehe z.B. Bunjes et al. (Langmuir, 1997, 13, 6188-6194) werden die mit einer DPTL-Monoschicht beschichteten Substrate in einer Pufferlösung von KCI 0,1 mol/l, Tricine 0,01 mol/l, Na₂HPO₄ 0,01 mol/l, MgSO₄ 0,0002 mol/l, in Wasser, pH = 7,4, geflutet. Dann wird die Schichtdicke mittels SPR, die Kapazität und der Widerstand der Monoschicht mittels IS gemessen. Die Temperatur in der Messzelle beträgt 32°C. Danach gibt man in die Messzelle von 1 ml Inhalt 50 µL einer Suspension von frisch durch Extrusion (durch ein 50 nm Filter) hergestellten Liposomen aus Diphytanoylphosphatidylcholin (DPhyPC) (4 mg/ml des o.g. Puffers). Die Schichtdickenzunahme wird durch SPR in Abhängigkeit von der Zeit beobachtet. Nach Erreichen eines stationären Zustandes und Spülen mit reiner Pufferlösung werden die Schichtdicke durch SPR sowie die elektrischen Eigenschaften durch IS gemessen.

Die Ergebnisse dieser Messungen sind in Tabelle 1 zusammengefasst, zum Vergleich sind darin auch die für die BLM bekannten Werte angegeben.

**Tabelle 1**

| | Cₘ/µF cm⁻² gemessen | Rₘ/MΩ cm² gemessen | Cₘ/µF cm⁻² BLM | Rₘ/MΩ cm² BLM | d/nm gemessen | d/nm berechnet |
|---|---|---|---|---|---|---|
| DPTL, Monoschicht Vor Vesikelfusion | 0.5 | 2.5 | | | 4.7 | 4.7 |
| Lipiddoppelschicht Nach Vesikelfusion | 0.42 | 6.2 | 0.5 | 10 | 8.5 | 8 |

Die Kinetik der Vesikelfusion mittels SPR ist in Abb. 5 und die SPR und IS Spektren vor und nach Vesikelfusion sind in Abb. 6 und 7 gezeigt.

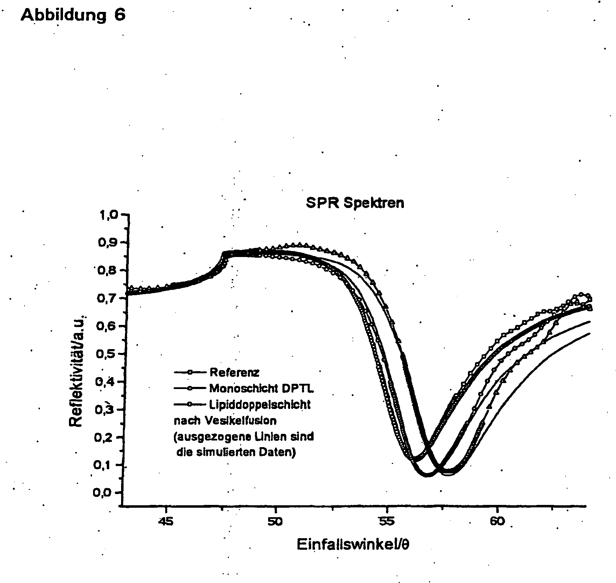

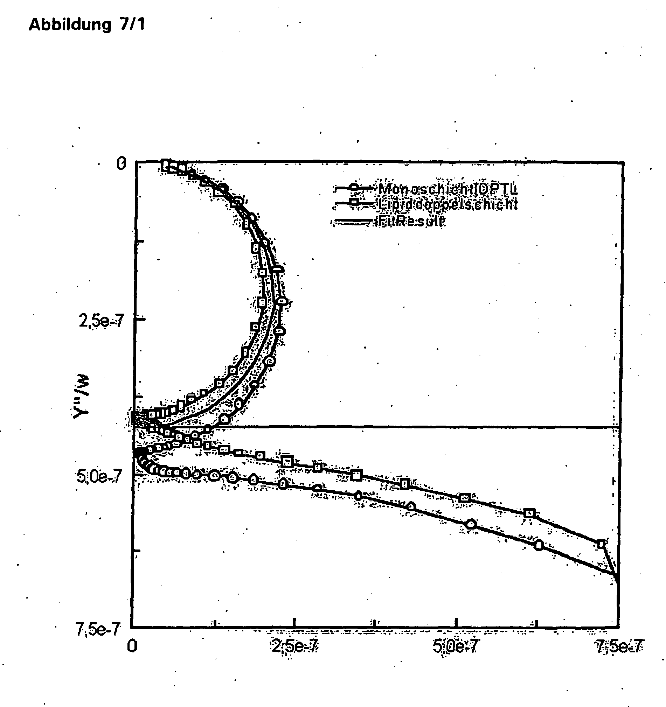

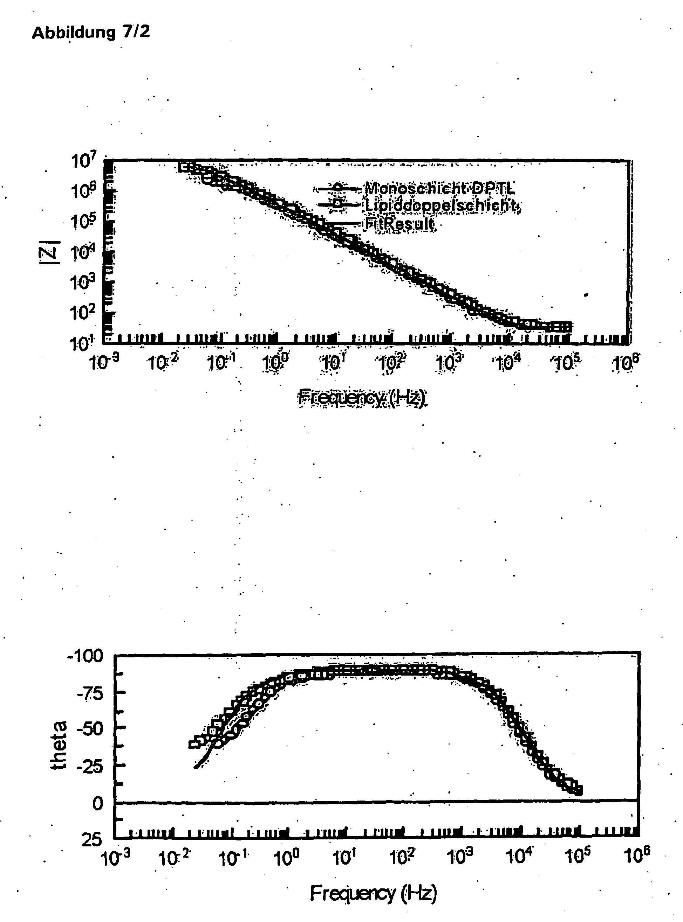

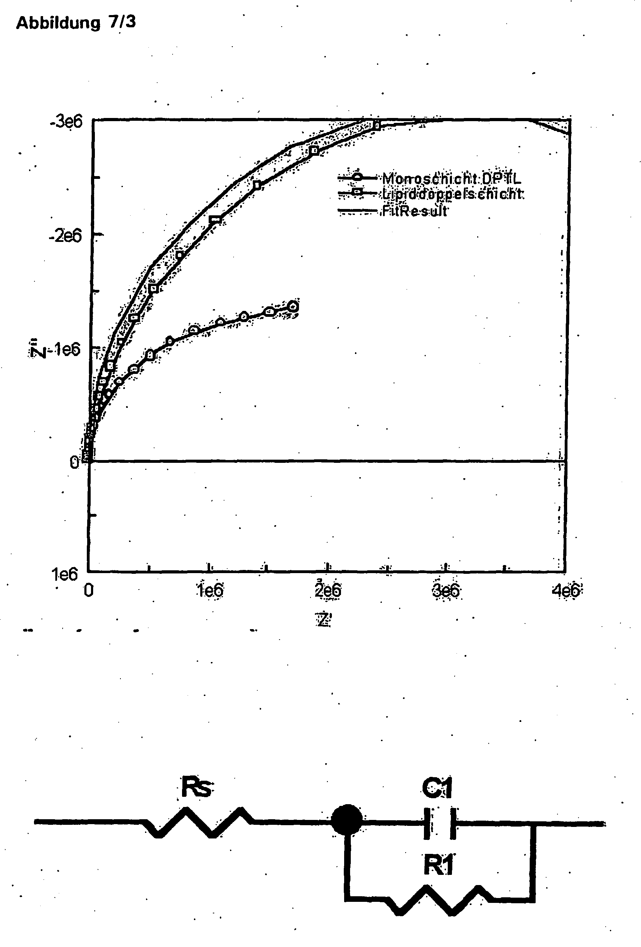

Ein Vergleich mit simulierten Daten (siehe ebenfalls Abbildung 6 und 7) zeigt die Einheitlichkeit der Schichten. Der Vergleich mit den elektrischen Daten von reinen Lipiddoppelschichten (Bilayer lipid membranes (BML's)) bzw. mit den aus den Moleküldimensionen berechneten Schichtdicken zeigt eine gute Übereinstimmung. Der Vergleich mit Literaturangaben über ebenfalls durch Vesikelfunktion hergestellte verankerte Lipiddoppelschichten auf Gold zeigt die wesentlich bessere Übereinstimmung der hier beschriebenen Schichten aus DPLT, mit BLM-Daten (mit biologischen Membranen vergleichbare Membranen werden in der Literatur mit einem Membranwiderstand von mehr als 10 MΩ cm² angegeben (Sackmann, Wagner, J. Phys. Chem. B, 2002)), besonders im Hinblick auf den Widerstand.

### Beispiel 5: Funktionalisierung einer verankerten Lipiddoppelschicht mit Valinomycin

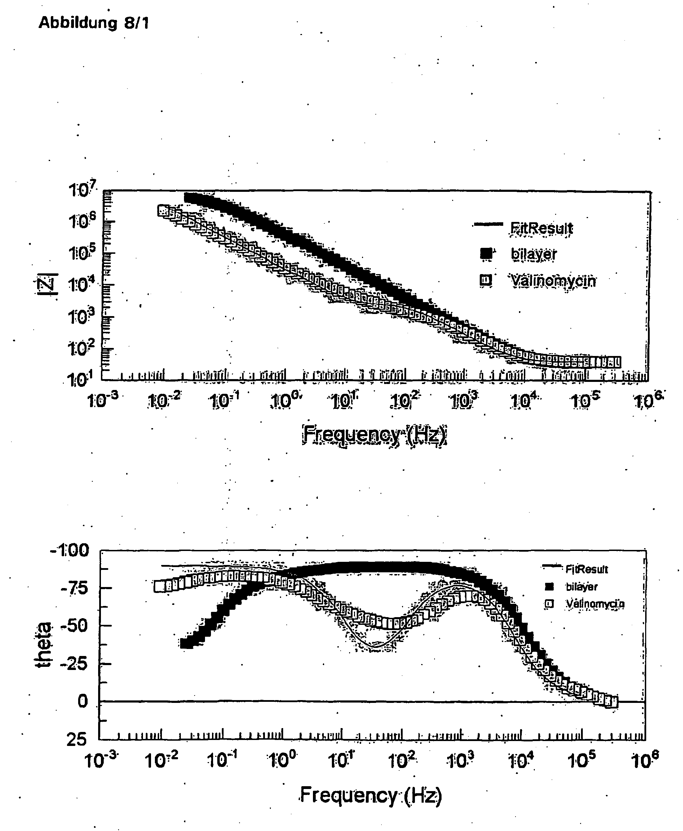

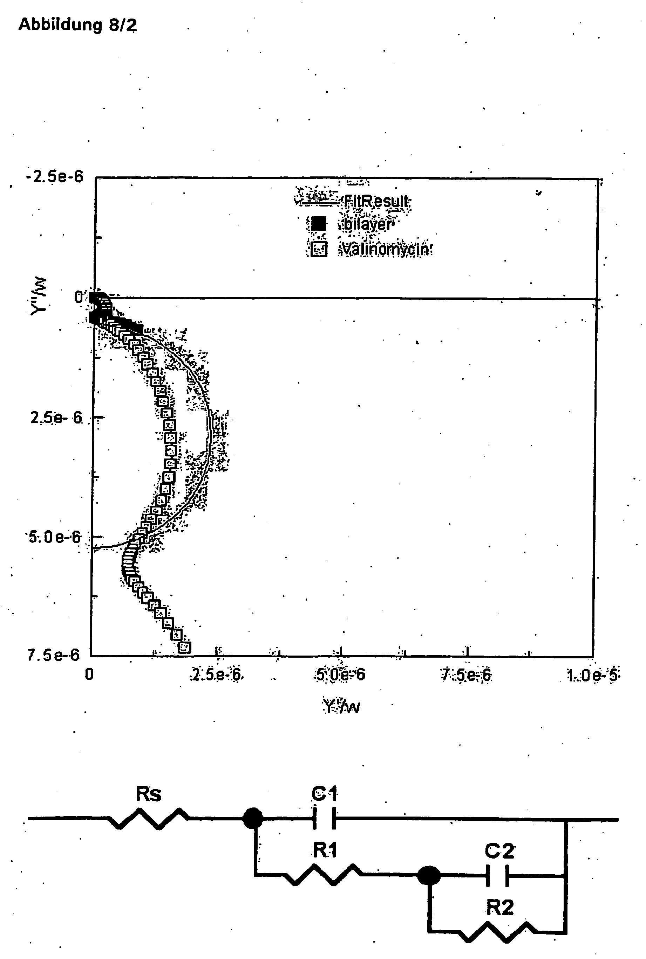

Zu der wie oben beschriebenen hergestellten Lipiddoppelschicht wird in der 1 ml Messzelle und in der o.g. Pufferlösung 50 µl einer Valinomycinlösung (8 x 10⁻⁵ M, in Ethanol) zugesetzt. Die IS-Spektren vor und nach dem Einbau des Valinomycins sind in Abb. 8 zusammen mit den an Ersatzschaltkreis angepassten Daten gezeigt. Kapazität und Widerstand aus dem angepaßen Daten sind in Tabelle 2 zusammengefasst.

**Tabelle 2:**

| | Cₘ/µF cm⁻² gemessen | Rₘ/MΩ cm² gemessen | Cₘ/µF cm⁻² BLM | Rₘ/MΩ cm² BLM | d/nm gemessen | d/nm berechnet |
|---|---|---|---|---|---|---|
| DPTL, Monoschicht Vor Vesikelfusion | 0.5 | 2.5 | | | 4.7 | 4.7 |
| Lipiddoppelschicht Nach Vesikelfusion | 0.42 | 6.2 | 0.5 | 10 | 8.5 | 8 |

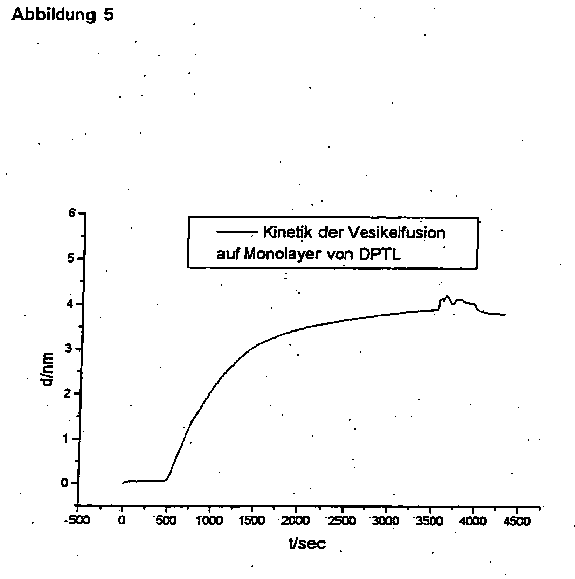

Die Kinetik der Vesikelfusion mittels SPR ist in Abb. 5 und die SPR und IS Spektren vor und nach Vesikelfusion sind in Abb. 6 und 7 gezeigt.

Nach dem Einbau von Valinomycin fällt (in Gegenwart der in der Pufferlösung vorhandenen Kaliumionen) der Widerstand der Schicht um 3 Größenordnungen. Außerdem ist die Kapazität der Ankerregion mit 4,6 µF cm⁻² erstens hinreichend verschieden von der Kapazität der Lipidschicht (dadurch ergibt sich ein deutlicher ,inflection point' im IS-Spektrum) und zweitens sehr gut vergleichbar mit den in der Literatur beschriebenen, aber mit größerem Aufwand hergestellten verankerten Lipidschichten von Cornell et al (supra).

## Patentansprüche

1. Verbindungen der allgemeinen Formeln (Ia) oder (Ib) 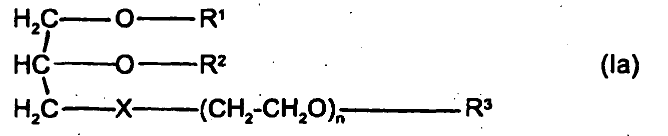 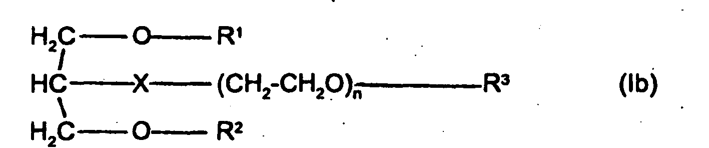
worin mindestens einer von R¹ und R² jeweils unabhängig ein gesättigter oder ungesättigter Kohlenwasserstoffrest oder ein Acylrest mit einer Kettenlänge von 10-22 C-Atomen ist, der gegebenenfalls durch eine oder mehrere Seitengruppen, substituiert sein kann, und der andere Wasserstoff, ein C₁-C₉-Kohlenwasserstoffrest oder ein Rest, umfassend eine Phospholipid-, Carboxyl-, Carbonyl-, SO-, SO₂-, Amid-, Amino- oder Thiolgruppe mit oder ohne einem C₁-C₉- Kohlenwasserstoffrest ist,
n eine ganze Zahl von 1-100 ist,
R³ ein eine Stützungsgruppe enthaltender Rest ist und
X O, S oder NR ist, wobei R H oder ein C₁-C₄-Alkylrest ist.

2. Verbindungen nach Anspruch 1,
**dadurch gekennzeichnet,**
**dass** zumindest einer von R¹ und R² ausgewählt ist aus gesättigten und ungesättigten Kohlenwasserstoffresten.

3. Verbindungen nach Anspruch 2,
**dadurch gekennzeichnet,**
**dass** zumindest einer von R¹ rund R² ausgewählt ist aus gesättigten Kohlenwasserstoffresten, die durch eine oder mehrere Methylgruppen substituiert sind.

4. Verbindungen nach einem der Ansprüche 1 bis 3,
**dadurch gekennzeichnet,**
**dass** X für O steht.

5. Verbindungen nach einem der Ansprüche 1 bis 4, **dadurch gekennzeichnet,**
**dass** n eine ganze Zahl von 2-70 ist.

6. Verbindungen nach einem der Ansprüche 1 bis 5,
**dadurch gekennzeichnet,**
**dass** die Stützungsgruppe ausgewählt ist aus S-haltigen Gruppen, Phosphingruppen und CN-Gruppen.

7. Verbindungen nach Anspruch 6,
**dadurch gekennzeichnet,**
**dass** die Stützungsgruppe eine S-S-Gruppe ist.

8. Verbindungen nach Anspruch 7,
**dadurch gekennzeichnet,**
**dass** die Stützungsgruppe eine cyclische S-S-Gruppe ist.

9. Verbindungen nach einem der Ansprüche 1 bis 8,
**dadurch gekennzeichnet,**
**dass** die Stützungsgruppe einen Liponsäurerest darstellt.

10. Verbindungen nach einem der Ansprüche 1 bis 9,
**dadurch gekennzeichnet,**
**dass** sie aus 2,3-Di-O-phytanyl-sn-glycero-1-tetraethylenglycol-DL-α-liponsäureester, 2,3-Di-O-phytanoyl-sn-glycero-1-tetraethylen-glycol-DL-a-liponsäureester, entsprechenden 1,2- oder 1,3-Diphytanyl- oder DiphytanoylDerivaten und optischen Isomeren davon ausgewählt ist.

11. Gestützte biomimetische Membran, umfassend eine Lipidschicht mit mindestens einer Verbindung der allgemeinen Formel (I) nach einem der Ansprüche 1 bis 10
als Stützungslipid zur Verankerung auf einem Substrat.

12. Gestützte Membran nach Anspruch 11,
**dadurch gekennzeichnet,**
**dass** das Substrat eine elektrisch leitfähige Oberfläche aufweist.

13. Gestützte Membran nach Anspruch 12,
**dadurch gekennzeichnet,**
**dass** das Substrat eine Edelmetalloberfläche aufweist.

14. Gestützte Membran nach Anspruch 13,
**dadurch gekennzeichnet,**
**dass** das Substrat eine Goldoberfläche aufweist.

15. Gestützte Membran nach einem der Ansprüche 11 bis 14,
**dadurch gekennzeichnet,**
**dass** das Substrat eine ultraglatte Oberfläche aufweist.

16. Gestützte Membran nach einem der Ansprüche 11 bis 15,
**dadurch gekennzeichnet,**
**dass** zusätzlich auf dem Substrat Spacermoleküle gebunden sind.

17. Gestützte Membran nach einem der Ansprüche 11 bis 16 weiterhin umfassend mindestens ein mobiles Lipid.

18. Gestützte Membran nach Anspruch 17,
**dadurch gekennzeichnet,**
**dass** das mobile Lipid ein Phospholipid oder ein nicht phosphorhaltiges Lipid versehen mit gleichen oder unterschiedlichen substituierten oder nichtsubstituierten Alkylketten mit 8-22 Kohlenstoffatomen, welche an Glycerin über Ether- oder/und Esterbindungen verknüpft sind, ein Glycolipid, eine quaternäre Ammoniumverbindung mit zwei C₁-C₄-Alkylgruppen und zwei C₈-C₁₈-Alkylketten, ein tertiäres Amin mit zwei C₈-C₁₈-Alkylketten und einer kurzen C₁-C₄-Alkylkette oder einem Wasserstoffatom, ein Steroid oder eine chemisch ähnliche Verbindung, welche vom Cholestangerüst abgeleitet ist, oder ein Isoprenoid sowie Lipidmischungen bestehend aus mehreren der genannten Verbindungen ist, wobei die Lipide gegebenenfalls polare Kopfgruppen, wie Hydroxyl, Carboxyl, Phosphorsäureester und deren Derivate, Schwefeloxidderivate mit unterschiedlicher Oxidationsstufe des Schwefels, Choline, Ethanolamine, Inositole, Kohlenhydrate, Glycerine, Aminoglycerine (in Sphingosinen) und Aminosäuren und deren neutrale, kationische oder anionische Formen oder Derivate enthalten können.

19. Gestützte Membran nach Anspruch 18,
**dadurch gekennzeichnet,**
**dass** die Lipidschicht als mobiles Lipid ausgewählt ist aus 1,2-Di-Ophytanyl-snglycerophosphocholin, 1,2-Di-O-phytanoyl-snglycerophosphocholin, 1,2-Di-O-phytanyl-sn-glycerophosphoethanolamin, 1,2-Di-O-phytanoyl-sn-glycerophosphoethanolamin sowie entsprechenden 1,2- oder 2,3-Diphytanyl- oder Diphytanoylderivaten sowie Mischungen von mehreren der genannten Verbindungen.

20. Gestützte Membran nach Anspruch 19,
**dadurch gekennzeichnet,**
**dass** die Lipidschicht als mobiles Lipid 1,2-Di-O-phytanoyl-sn-glycero-phosphocholin enthält.

21. Gestützte Membran nach einem der Ansprüche 15 bis 20,
**dadurch gekennzeichnet,**
**dass** sie eine Kapazität von 0,4 bis 0,7 µF/cm² aufweist.

22. Gestützte Membran nach einem der Ansprüche 15 bis 21,
**dadurch gekennzeichnet,**
**dass** sie einen Widerstand von mindestens 1 MΩ·cm² aufweist.

23. Gestützte Membran nach einem der Ansprüche 11 bis 22.
**dadurch gekennzeichnet,**
**dass** funktionelle Moleküle in die Lipidschicht eingebaut sind.

24. Gestützte Membran nach Anspruch 23,
**dadurch gekennzeichnet,**
**dass** die funktionellen Moleküle ausgewählt sind aus Proteinen, Antibiotika, Signalerkennungsmolekülen, Botenstoffen, Porinen, lonenkanälen, membranverändernden Substanzen, wie z.B. Narkotika, Carriern, Liganden, Rezeptoren, Peptiden, Glycolipiden und herterozyklischen Verbindungen.

25. Verwendung einer gestützten Membran nach einem der Ansprüche 11 bis 24 als Biosensor.

26. Verwendung nach Anspruch 25 in einem Nachweisverfahren.

27. Verwendung nach Anspruch 25 in einem Screeningverfahren.

28. Verwendung nach Anspruch 27 zur Identifizierung oder/und Charakterisierung pharmakologischer Wirkstoffe.

29. Verwendung nach Anspruch 25 zur Analyse von Biomolekülen.

30. Verwendung nach Anspruch 29 zur Analyse von membranassoziierten Biomolekülen, ausgewählt aus Proteinen, Antibiotika, Signalerkennungsmolekülen, Botenstoffen, Porinen, lonenkanälen, membranverändernden Substanzen, wie z.B. Narkotika, Carriern, Liganden, Rezeptoren, Peptiden, Glycolipiden und herterozyklischen Verbindungen.

31. Verwendung nach Anspruch 25 als elektrische Dichtungsmaterialien.

## Claims

1. Compounds of general formulae (Ia) or (Ib) 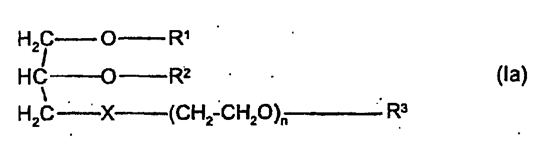 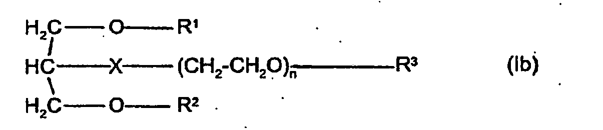 wherein at least one of R¹ and R², independently in each case, is a saturated or unsaturated hydrocarbon radical or an acyl radical which has a chain length of 10-22 C atoms and which can optionally be substituted by one or more side groups, and the other is hydrogen, a C₁-C₉ hydrocarbon radical or a radical comprising a phospholipid, carboxyl, carbonyl, SO, SO₂, amide, amino or thiol group with or without a C₁-C₉ hydrocarbon radical,
n is an integer from 1 to 100,
R³ is a radical containing a tethering group and
X is O, S or NR, R being H or a C₁-C₄ alkyl radical.

2. Compounds according to claim 1, **characterised in that** at least one of R¹ and R² is selected from saturated and unsaturated hydrocarbon radicals.

3. Compounds according to claim 2, **characterised in that** at least one of R¹ and R² is selected from saturated hydrocarbon radicals which are substituted by one or more methyl groups.

4. Compounds according to any one of claims 1 to 3, **characterised in that** X represents O.

5. Compounds according to any one of claims 1 to 4, **characterised in that** n is an integer from 2 to 70.

6. Compounds according to any one of claims 1 to 5, **characterised in that** the tethering group is selected from S-containing groups, phosphine groups and CN groups.

7. Compounds according to claim 6, **characterised in that** the tethering group is an S-S group.

8. Compounds according to claim 7, **characterised in that** the tethering group is a cyclic S-S group.

9. Compounds according to any one of claims 1 to 8, **characterised in that** the tethering group represents a lipoic acid radical.

10. Compounds according to any one of claims 1 to 9, **characterised in that** they are selected from 2,3-di-O-phytanyl-sn-glycero-1-tetraethyleneglycol-DL-α-lipoic acid ester, 2,3-di-O-phytanoyl-sn-glycero-1-tetraethyleneglycol-DL-α-lipoic acid ester, corresponding 1,2- or 1,3-diphytanyl or diphytanoyl derivatives, and optical isomers thereof.

11. Supported biomimetic membrane, comprising a lipid layer containing at least one compound of general formula (I) according to any one of claims 1 to 10 as a tethering lipid for anchoring to a substrate.

12. Supported membrane according to claim 11, **characterised in that** the substrate has an electrically conductive surface.

13. Supported membrane according to claim 12, **characterised in that** the substrate has a noble metal surface.

14. Supported membrane according to claim 13, **characterised in that** the substrate has a gold surface.

15. Supported membrane according to any one of claims 11 to 14, **characterised in that** the substrate has an ultra-smooth surface.

16. Supported membrane according to any one of claims 11 to 15, **characterised in that** spacer molecules are additionally bound to the substrate.

17. Supported membrane according to any one of claims 11 to 16, further comprising at least one mobile lipid.

18. Supported membrane according to claim 17, **characterised in that** the mobile lipid is a phospholipid or a lipid which does not contain phosphorus, provided with identical or different substituted or unsubstituted alkyl chains with 8-22 carbon atoms which are linked to glycerol via ether or/and ester bonds, a glycolipid, a quartemary ammonium compound containing two C₁-C₄ alkyl groups and two C₈-C₁₈ alkyl chains, a tertiary amine containing two C₈-C₁₈ alkyl chains and a short C₁-C₄ alkyl chain or a hydrogen atom, a steroid or a chemically similar compound which is derived from the cholestane structure, or an isoprenoid as well as lipid mixtures consisting of a plurality of said compounds, the lipids optionally containing polar head groups such as hydroxyl, carboxyl, phosphoric acid ester and derivatives thereof, sulphur oxide derivatives with different oxidation stages of the sulphur, cholines, ethanolamines, inositols, carbohydrates, glycerols, aminoglycerols (in sphingosines) and amino acids and neutral, cationic or anionic forms or derivatives thereof.

19. Supported membrane according to claim 18, **characterised in that** the mobile lipid of the lipid layer is selected from 1,2-di-O-phytanyl-sn-glycerophosphocholine, 1,2-di-O-phytanoyl-sn-glycerophosphocholine, 1,2-di-O-phytanyl-sn-glycerophosphoethanolamine, 1,2-di-O-phytanoyl-sn-glycerophosphoethanolaminc and corresponding 1,2- or 2,3-diphytanyl or diphytanoyl derivatives, and mixtures of a plurality of said compounds.

20. Supported membrane according to claim 19, **characterised in that** the lipid layer contains 1,2-di-O-phytanoyl-sn-glycero-phosphocholine as the mobile lipid.

21. Supported membrane according to any one of claims 15 to 20, **characterised in that** it has a capacitance of 0.4 to 0.7 µF/cm².

22. Supported membrane according to any one of claims 15 to 21, **characterised in that** it has a resistance of at least 1 MΩ·cm².

23. Supported membrane according to any one of claims 11 to 22, **characterised in that** functional molecules are incorporated into the lipid layer.

24. Supported membrane according to claim 23, **characterised in that** the functional molecules are selected from proteins, antibiotics, signal recognition molecules, messenger substances, porines, ion channels, membrane-changing substances such as narcotics, carriers, ligands, receptors, peptides, glycolipids and heterocyclic compounds.

25. Use of a supported membrane according to any one of claims 11 to 24 as a biosensor.

26. Use according to claim 25 in a detection method.

27. Use according to claim 25 in a screening method.

28. Use according to claim 27 to identify or/and characterise pharmacologically active substances.

29. Use according to claim 25 to analyse biomolecules.

30. Use according to claim 29 for the analysis of membrane-associated biomolecules selected from proteins, antibiotics, signal recognition molecules, messenger substances, porines, ion channels, membrane-changing substances such as narcotics, carriers, ligands, receptors, peptides, glycolipids and heterocyclic compounds.

31. Use according to claim 25 as electrical insulation materials.

## Revendications

1. Composés de formule générale (Ia) ou (Ib) 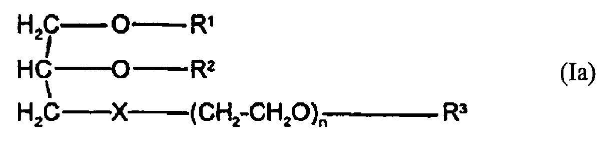 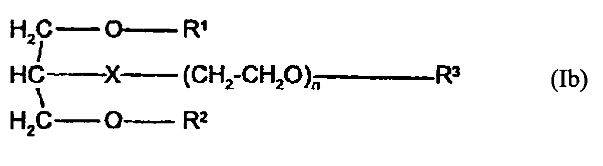 dans laquelle
l'un au moins de R¹ et R² est indépendamment de l'autre un reste hydrocarbure saturé ou insaturé ou un reste acyle ayant une longueur de chaîne de 10 à 22 atomes de carbone qui peut être substitué le cas échéant par un ou plusieurs groupes latéraux, et l'autre est un hydrogène, un reste hydrocarbure en C₁ à C₉ ou un reste comprenant un groupe phospholipide, carboxyle, carbonyle, SO-, SO₂-, amido, amino ou thiol avec ou sans reste hydrocarbure en C₁ à C₉,
n est un nombre entier valant de 1 à 100,
R³ est un reste contenant un groupe de support, et
X est O, S ou NR, où R est H ou un reste alkyle en C₁ à C₄.

2. Composés selon la revendication 1, **caractérisés en ce que** l'un au moins de R¹ et R² est choisi parmi des restes hydrocarbure saturés et insaturés.

3. Composés selon la revendication 2, **caractérisés en ce que** l'un au moins de R¹ et R² est choisi parmi des restes hydrocarbure saturés qui sont substitués par un ou plusieurs groupes méthyle.

4. Composés selon l'une des revendications 1 à 3, **caractérisés en ce que** X représente O.

5. Composés selon l'une des revendications 1 à 4, **caractérisés en ce que** n est un nombre entier valant de 2 à 70.

6. Composés selon l'une des revendications 1 à 5, **caractérisés en ce que** le groupe de support est choisi parmi des groupes contenant du S, des groupes phosphine et des groupes CN.

7. Composés selon la revendication 6, **caractérisés en ce que** le groupe de support est un groupe S-S.

8. Composés selon la revendication 7, **caractérisés en ce que** le groupe de support est un groupe S-S cyclique.

9. Composés selon l'une des revendications 1 à 8, **caractérisés en ce que** le groupe de support est un reste acide lipoïque.

10. Composés selon l'une des revendications 1 à 9, **caractérisés en ce qu'**ils sont choisis parmi le DL-α-lipoate de 2,3-di-O-phytanyl-sn-glycéro-1-tétraéthylène glycol, le DL-α-lipoate de 2,3-di-O-phytanoyl-sn-glycéro-1-tétraéthylène glycol, les dérivés de 1,2- ou 1,3-diphytanyle ou diphytanoyle ainsi que leurs isomères optiques.

11. Membrane biomimétique supportée, comprenant une couche lipidique avec au moins un composé de formule générale (I) selon l'une des revendications 1 à 10 en tant que lipide de support pour l'ancrage à un substrat.

12. Membrane supportée selon la revendication 11, **caractérisée en ce que** le substrat présente une surface électriquement conductrice.

13. Membrane supportée selon la revendication 12, **caractérisée en ce que** le substrat présente une surface en métal noble.

14. Membrane supportée selon la revendication 13, **caractérisée en ce que** le substrat présente une surface en or.

15. Membrane supportée selon l'une des revendications 11 à 14, **caractérisée en ce que** le substrat présente une surface ultra-lisse.

16. Membrane supportée selon l'une des revendications 11 à 15, **caractérisée en ce que** des molécules espaceuses sont liées en plus au substrat.

17. Membrane supportée selon l'une des revendications 11 à 16, comprenant en plus au moins un lipide mobile.

18. Membrane supportée selon la revendication 17, **caractérisée en ce que** le lipide mobile est un phospholipide ou un lipide non phosphoré doté de chaînes alkyle identiques ou différentes, substituées ou non substituées, de 8 à 22 atomes de carbone qui sont reliées au glycérol par le biais de ponts éther ou/et ester, un glycolipide, un composé d'ammonium quaternaire avec deux groupes alkyle en C₁ à C₄ et deux chaînes alkyle en C₈ à C₁₈, une amine tertiaire avec deux chaînes alkyle en C₈ à C₁₈ et une courte chaîne alkyle en C₁ à C₄ ou un atome d'hydrogène, un stéroïde ou un composé chimiquement similaire dérivé du squelette cholestane, ou un isoprénoïde, ainsi que des mélanges lipidiques composés de plusieurs des composés précités, les lipides pouvant contenir le cas échéant des groupes de tête polaires tels que des groupes hydroxyle, carboxyle, des esters de l'acide phosphorique et leurs dérivés, des dérivés de l'oxyde de soufre avec différents degrés d'oxydation du soufre, des cholines, des éthanolamines, des inositols, des hydrates de carbone, des glycérols, des aminoglycérols (dans les sphingosines) et des acides aminés ainsi que leurs formes neutres, cationiques ou anioniques ou leurs dérivés.

19. Membrane supportée selon la revendication 18, **caractérisée en ce que** la couche lipidique servant de lipide mobile est choisie parmi la 1,2-di-O-phytanyl-sn-glycérophosphocholine, la 1,2-di-O-phytanoyl-sn-glycéro-phosphocholine, la 1,2-di-O-phytanyl-sn-glycérophospho-éthanolamine, la 1,2-di-O-phytanoyl-sn-glycérophospho-éthanolamine ainsi que les dérivés de 1,2- ou 2,3-diphytanyle ou diphytanoyle correspondants, de même que des mélanges de plusieurs des composés cités.

20. Membrane supportée selon la revendication 19, **caractérisée en ce que** la couche lipidique servant de lipide mobile contient de la 1,2-di-O-phytanoyl-sn-glycérophosphocholine.

21. Membrane supportée selon l'une des revendications 15 à 20, **caractérisée en ce qu'**elle présente une capacité de 0,4 à 0,7 µF/cm².

22. Membrane supportée selon l'une des revendications 15 à 21, **caractérisée en ce qu'**elle présente une résistance d'au moins 1 MΩ·cm².

23. Membrane supportée selon l'une des revendications 11 à 22, **caractérisée en ce que** des molécules fonctionnelles sont incorporées dans la couche lipidique.

24. Membrane supportée selon la revendication 23, **caractérisée en ce que** les molécules fonctionnelles sont choisies parmi des protéines, des antibiotiques, des molécules de reconnaissance de signal, des substances messagères, des porines, des canaux ioniques, des substances modifiant la membrane telles que des narcotiques, des véhicules, des ligands, des récepteurs, des peptides, des glycolipides ainsi que des composés hétérocycliques.

25. Utilisation d'une membrane supportée selon l'une des revendications 11 à 24 en tant que biocapteur.

26. Utilisation selon la revendication 25 dans un procédé de dosage.

27. Utilisation selon la revendication 25 dans un procédé de criblage.

28. Utilisation selon la revendication 27 pour identifier ou/et caractériser des substances actives pharmacologiques.

29. Utilisation selon la revendication 25 pour l'analyse de biomolécules.

30. Utilisation selon la revendication 29 pour l'analyse de biomolécules associées à la membrane, choisies parmi des protéines, des antibiotiques, des molécules de reconnaissance de signal, des substances messagères, des porines, des canaux ioniques, des substances modifiant la membrane telles que des narcotiques, des véhicules, des ligands, des récepteurs, des peptides, des glycolipides ainsi que des composés hétérocycliques.

31. Utilisation selon la revendication 25 comme matériau d'étanchéité électrique.
